# EUROPEAN PATENT SPECIFICATION

(11) **EP 2 697 647 B1**
(45) Date of publication and mention of the grant of the patent: **30.08.2017**
(21) Application number: 12717075.1
(22) Date of filing: 13.04.2012
(51) Int. Cl.: G01N 33/53, G01N 33/543

(54) **ASSAY METHOD FOR INTRINSIC ACUTE KIDNEY INJURY**
TESTVERFAHREN FÜR INTRINSISCH AKUTE NIERENVERLETZUNG
PROCÉDÉ DE DOSAGE POUR LES AFFECTIONS RÉNALES AIGUËS INTRINSÈQUES

(30) Priority: 13.04.2011 EP 11162344
(43) Date of publication of application: 19.02.2014
(73) Proprietor: Charité - Universitätsmedizin Berlin, 10117 Berlin (DE)
(72) Inventor: WESTHOFF, Timm, H., 12159 Berlin (DE); HELLER, Frank, 10435 Berlin (DE); ARMBRUSTER, Franz Paul, 64625 Bensheim (DE)
(74) Representative: Schulz Junghans Patentanwälte PartGmbB
(86) International application number: PCT/EP2012/056754
(87) International publication number: WO 2012/140187

(56) References cited:
- US-A1- 2010 233 740
- MIKLASZEWSKA MONIKA ET AL: "[Early laboratory markers of acute renal failure].", PRZEGLAD LEKARSKI 2006 LNKD- PUBMED:16967714, vol. 63, no. 2, 2006, pages 81-84, XP009150653, ISSN: 0033-2240
- ELKJAER M ET AL: "A new rapid home test for faecal calprotectin in ulcerative colitis.", ALIMENTARY PHARMACOLOGY & THERAPEUTICS 15 JAN 2010 LNKD- PUBMED:19817723, vol. 31, no. 2, 15 January 2010 (2010-01-15), pages 323-330, XP002654873, ISSN: 1365-2036
- ALON U S ET AL: "Assessment and interpretation of urinalysis and routine kidney function tests - Part II: Kidney function tests", CHILDREN'S HOSPITAL QUARTERLY 1992 US, vol. 4, no. 4, 1992, pages 213-220, XP009150650, ISSN: 0899-5869
- MALÍCKOVÁ KARIN ET AL: "Plasma calprotectin in chronically dialyzed end-stage renal disease patients.", INFLAMMATION RESEARCH : OFFICIAL JOURNAL OF THE EUROPEAN HISTAMINE RESEARCH SOCIETY ... [ET AL.] APR 2010 LNKD- PUBMED:19856199, vol. 59, no. 4, April 2010 (2010-04), pages 299-305, XP002654874, ISSN: 1420-908X
- ZHANG PING L ET AL: "Heat shock protein expression is highly sensitive to ischemia-reperfusion injury in rat kidneys", ANNALS OF CLINICAL AND LABORATORY SCIENCE, vol. 38, no. 1, January 2008 (2008-01), pages 57-64, XP002654875, ISSN: 0091-7370
- SZETO CHEUK-CHUN ET AL: "Urinary expression of kidney injury markers in renal transplant recipients.", CLINICAL JOURNAL OF THE AMERICAN SOCIETY OF NEPHROLOGY : CJASN DEC 2010 LNKD- PUBMED:20671224, vol. 5, no. 12, December 2010 (2010-12), pages 2329-2337, XP009150652, ISSN: 1555-905X
- DU CHEYRON DAMIEN ET AL: "Urinary measurement of Na+/H+ exchanger isoform 3 (NHE3) protein as new marker of tubule injury in critically ill patients with ARF.", AMERICAN JOURNAL OF KIDNEY DISEASES : THE OFFICIAL JOURNAL OF THE NATIONAL KIDNEY FOUNDATION SEP 2003 LNKD- PUBMED:12955677, vol. 42, no. 3, September 2003 (2003-09), pages 497-506, XP002655116, ISSN: 1523-6838

## Description

### TECHNICAL FIELD

The present invention relates to assay methods for acute kidney injury (AKI).

### STATE OF THE ART

Acute kidney injury (AKI) or acute renal failure (ARF) is a rapid loss of kidney function. Its causes are numerous and include low blood volume, exposure to toxins, and urinary obstruction (e.g. prostate enlargement). AKI is diagnosed on the basis of clinical history, such as decreased urine production, and characteristic laboratory findings, such as elevated blood urea nitrogen and serum creatinine. Depending on its severity, AKI may lead to a number of complications, including metabolic acidosis, high potassium levels, uraemia, changes in body fluid balance, and effects to other organ systems. Management includes supportive care, such as renal replacement therapy, as well as treatment of the underlying disorder. A kidney injury is common among hospitalised patients. It affects 1-5 % of all hospitalised patients and approximately 25 to 30 percent of patients in the intensive care unit. The prognosis of AKI crucially depends on an early diagnosis and an immediate onset of therapy. The earlier the cause of AKI is identified, the more successful is the specific therapy.

However, a myriad of causes may lead to AKI, which are commonly classified according to their origin into prerenal, intrinsic (intrarenal), and postrenal. Prerenal causes of AKI are those that decrease effective blood flow to the kidney and they stand for a loss of renal function in spite of intact nephrons. The major causes of prerenal AKI are low blood volume, hypotension and heart failure as well as local changes to the blood vessels supplying the kidney such as renal artery stenosis or renal vein thrombosis or renal ischaemia. There is a broad spectrum of intrarenal causes of AKI including acute tubular necrosis (ATN), interstitial nephritis (AIN), glomerulonephritis and vasculitis. Postrenal AKI is a consequence of urinary tract obstruction. This may be related to benign prostatic hyperplasia, kidney stones, obstructed urinary catheter, bladder stone, etc.. The diagnosis of postrenal AKI may be established by ultrasound.

The two major causes of AKI developing in the hospital are prerenal disease and acute tubular necrosis (ATN) which belongs to the intrarenal causes of AKI (intrinsic AKI). Together, they account for approximately 70 to 75% of all causes of AKI.

Treatment of prerenal, postrenal and intrinsic AKI differs considerably. In prerenal AKI, therapy consists principally of correction of absolute of effective volume depletion. Postrenal AKI may be resolved simply by elimination of obstruction. The therapy of intrinsic AKI, however, depends on the specific diagnosis.

The diagnostic differentiation of pre- and intrarenal AKI, however, is difficult. To date, there is no reliable marker available for the detection of prerenal AKI. Response to fluid repletion is still regarded as the diagnostic test of choice to achieve differentiation between prerenal and intrinsic disease. Return of renal function to baseline within 24 to 72 hours is considered to indicate prerenal AKI, whereas persistent renal failure indicates intrinsic disease. This criterion is not valid, however, in cases of short-lived ATN. Furthermore, intrarenal causes such as crescentic glomerulonephritis require immediate biopsy in order to avoid delay in the initiation of therapy. Therefore, waiting for 72 hours before initiation of therapy is unacceptable. Finally, rapid fluid application is contraindicated in a substantial number of patients, such as those with congestive heart failure.

In addition to the fluid challenge there are laboratory parameters that are propagated for the differentiation of prerenal and intrinsic AKI. The most commonly used parameter is the fractional excretion of sodium (FE_{Na}). It is typically < 1% in prerenal disease (indicative of sodium retention) and > 2% in intrinsic disease (Steiner RW, Am. J. Med. 1984; 77(4): 699-702). There are several conditions, however, in which this distinction is not accurate: FE_{Na} may not be used in the presence of diuretics, since diuretics increase sodium excretion independent of the underlying cause of AKI. Furthermore, in the setting of reduced effective blood volume, such as congestive heart failure or liver cirrhosis, FE_{Na} is reduced, making its interpretation in AKI impossible. Hence, FE_{Na} cannot be used in the majority of elderly patients with pre-existing diseases.

Miklaszewska M et al discuss in Przeglad Lekarski (2006) 63:81-84 a number of laboratory markers of acute renal failure and to whether they allow differential diagnostics between acute prerenal and intrinsic acute renal failure. However, it was noted that further clinical research is required. The document states that differential diagnostics, especially between acute prerenal and intrinsic acute renal failure is an important stage in patient evaluation process. In the article the authors present a short and concise profile of novel diagnostic AKI biomarkers: neutrophil gelatinase associated lipocalin (NGAL), sodium/hydrogen exchanger isoform 3 (NHE3), human kidney injury molecule-1 (hKIM-1), interleukin 6 (IL-6), interleukin 8 (IL-8), interleukin 18 (IL-18), urinary cysteinerich protein (Cyr 61), urinary glutathione-S-transferase (GST), cystatin C, spermidine/spermine N-acetyl transferase (SSAT) and actin). Calprotectin is not mentioned as marker. Elkjaer et al. describe a rapid ELISA-based home test for faecal calprotectin in ulcerative colitis (Aliment Pharmacol Ther. 2010 Jan 15, 31(2):323-30). New quantitative rapid tests using a lateral flow device have been developed. Du Cheyron et al. disclose that urinary NHE3 abundance might be a novel noninvasive marker of renal tubule damage, helping to differentiate prerenal azotemia, ATN, and intrinsic ARF other than ATN (Am J Kidney Dis. 2003 Sep, 42(3):497-506).

With regard to the lack of a reliable non-invasive marker of prerenal and intrinsic AKI, a definite assignment to one of these entities may only be done by biopsy. A rapid non-invasive test for the differentiation of prerenal and intrinsic AKI is neither available nor known. A renal biopsy, however, bears the risk of complications - even in the hands of an experienced examiner. Despite any potential complications, a biopsy is usually unavoidable in the differential diagnosis of intrinsic AKI. It provides no further information, however, in the prerenal disease.

The state of the art therefore represents a problem.

### SUMMARY OF THE INVENTION

This problem is solved by an assay method for intrinsic acute kidney injury (intrarenal AKI) which encompasses the rapid assessment of the amount calprotectin in a urine sample of said patient. The method according to the invention comprises the steps of (i) taking a urine sample of a patient suspected of suffering from prerenal or intrarenal (intrinsic) AKI; (ii) determining the calprotectin level in the urine sample obtained in step (i); and (iii) assessing the urinary calprotectin level obtained in step (ii) to a threshold level, wherein a level higher than said threshold level indicates that the patient is suffering from intrinsic acute kidney injury and level lower than said threshold level indicates that the patent is suffering from prerenal kidney injury. Due to the clear separation of the urinary calprotectin levels for prerenal and intrinsic AKI, the assessment of the calprotectin level in the urine sample may further be done by lateral-flow immunochromatography so that the result is available within a few minutes rather than hours. More precisely, said patient may be diagnosed with intrinsic acute kidney injury if the concentration determined in step (ii) is higher than said threshold. Alternatively, said patient may be diagnosed with prerenal acute kidney injury if the concentration determined in step (ii) is lower than said threshold value, provided a post-renal acute kidney injury due to a urinary tract obstruction has been ruled out, e.g. by renal ultrasound.

The inventors have discovered that the concentration of urinary calprotectin represents an easily assessable parameter which allows rapid and clear differentiation between prerenal and intrinsic AKI and corresponding diagnoses. Whereas calprotectin levels in patients suffering from prerenal AKI are comparable to those in healthy subjects, high urinary calprotectin levels indicate the intrinsic disease.

In one embodiment of the invention, the method comprises the step of further determining the level of urinary creatinine in said urine sample of a patient suspected of suffering from prerenal or intrarenal (intrinsic) AKI and a calculation of the concentration by weight ratio of calprotectin to creatinine in said urine sample. This embodiment of the method of diagnosis is particularly preferred when the volume or the concentration of the urine sample has become altered in any way, e.g. by medical treatment of the patient. The method then comprises the steps of (i) taking a urine sample from a patient suspected of suffering from prerenal or intrinsic acute kidney injury; (ii) determining levels of calprotectin and creatinine in the urine sample obtained in step (i); (iii) calculating the concentration by weight ratio of urinary calprotectin to urinary creatinine in said sample; and (iv) comparing the obtained weight ratio of calprotectin and creatinine obtained in step (iii) to a threshold; and wherein the patient is diagnosed with intrinsic acute kidney injury if the ratio determined in step (iv) is higher than said threshold value, and the patient is diagnosed with prerenal acute kidney injury if the ratio determined measured in step (iv) is lower than said threshold value.

A diagnostically useful threshold level for urinary calprotectin lies in the range of 200 to 400 ng/ml, preferably close to 300 ng/ml. A cut-off or threshold level of 300 ng/ml provides a sensitivity of greater than 90% and a specificity of approximately 98%. A diagnostically useful threshold for the concentration by weight ratio of calprotectin to creatinine is in the range from 0.0003 to 0.0006, preferably close to 0.0005.

These threshold levels have been determined from the inventors' set of data. If ROC analysis is used to determine the optimal levels for the differentiation of prerenal and intrinsic AKI, the set of data below suggests a sensitivity of 92.3% and a specificity of 97.1% for a threshold level of 300 ng urinary calprotectin per millilitre. The resulting positive and negative predictive values are 98.0% and 89.2%, respectively. The urinary calprotectin/creatinine ratio achieved very similar accuracy parameters at a threshold level of 0.0005 (= 500 ng calprotectin/ml / grams of urinary creatinine /L). When all cases with urinary tract infection are excluded, the ROC curve's AUC is 0.948 for calprotectin and 0.949 for the calprotectin/creatinine ratio. Specificity is 97.1%, and sensitivity is 89.7% for the calprotectin/creatinine parameters. Accordingly, the positive predictive value is 96.3%, whereas the negative predictive value becomes to 91.7%.

The accuracy of urinary calprotectin in the differential diagnosis of AKI is therefore higher than the accuracy of any other established diagnostic marker. Whereas urinary calprotectin levels in prerenal disease are comparable to healthy controls, highly increased calprotectin concentrations in a urine sample of a patient allow a reliable diagnosis of intrinsic AKI. The large separation and discrete clustering of the measurement values for urinary calprotectin in cases of prerenal and intrinsic AKI suggest the use of an instant assay method such as a lateral flow immunochromatography to distinguish cases of prerenal and intrinsic AKI. Notwithstanding, it is still prudent to examine the respective patient for postrenal acute kidney injury, urinary tract infections and/or menstrual bleeding prior to therapy.

In one further embodiment of the invention the urinary calprotectin concentration is therefore assessed by means of a lateral-flow immunochromatography, preferably a lateral-flow immunoassay which detects urinary calprotectin in a urine sample, if it is present (or absent) above said threshold concentration. Another aspect of the invention therefore concerns the use of a lateral-flow immunoassay in a method of diagnosis of intrinsic acute kidney injury, wherein the staining reaction is calibrated to become visible if the urine sample comprises approximately or more than said threshold level, e.g. a level of 300 ng calprotectin per ml urine.

The lateral-flow immunoassay may be designed for point of care testing, preferably in a dipstick format. The dipstick format is a form of immunoassay in which the test sample flows along a solid substrate via capillary action. After the urine is applied to the test pad it encounters a coloured reagent (e.g. an anti-calprotectin antibody or antibody fragment conjugated to a marker or reporter molecule) which mixes with the sample and transits the substrate encountering lines or zones pre-treated with capture antibodies or antigen (which is described herein as "receptor". The receptor may be any binding molecule such as streptavidin or avidin, antibody and antibody fragment, aptamer, spiegelmer). If analyte (calprotectin) is present in the urine sample the coloured reagent will then be bound at the test line or zone and the test line or zone can be calibrated such that it becomes visually detectable, say visible. The lateral flow tests may operate as a competitive or a sandwich assay. The components, the coloured reagents or reporter molecules and the test line or zone of this lateral flow immunoassay can be calibrated by serial testing so that the staining of the test line or zone becomes visible when the urinary calprotectin concentration is close to or higher than the pre-determined threshold, e.g. higher than 300 ng/ml. Alternatively, it may also be possible to run in parallel lateral flow immunochromatographic tests on the test sample and a standard liquid comprising a threshold concentration of calprotectin, and comparing the colour reaction using a suitable device such as a Lateral Flow Reader. It is also possible to comprise a reference line or zone on the lateral flow test itself.

Also part of the present invention is the use of a lateral flow test in the detection of urinary calprotectin in a method for differentiating between intrinsic acute kidney injury and prerenal acute kidney injury.

Furthermore, a method of diagnosis of a patient having acute kidney injury comprises the steps described above, wherein, if said patient is diagnosed with intrinsic acute kidney injury, (a) a renal biopsy is taken from said patient and the cause of the diagnosed intrinsic acute kidney injury is determined using known methods, and (b) an appropriate therapy is initiated. Alternatively, if said patient is diagnosed with prerenal acute kidney injury, an appropriate therapy is initiated by correction of absolute or intrinsic volume depletion.

### BRIEF DESCRIPTION OF THE FIGURES

The invention will be presented and illustrated with the help of the following Figures:
- Fig. 1a: shows the urinary calprotectin measurements in the study population for control subjects, prerenal AKI patients and intrinsic AKI patients;
- Fig. 1b: shows the measurements of the urinary calprotectin/creatinine concentration ratios in the study population for control subjects, prerenal AKI patients and intrinsic AKI patients;
- Fig. 2a: shows the calprotectin measurements in the study population for subjects, prerenal AKI patients and intrinsic AKI patients after exclusion of patients with urinary tract infections;
- Fig. 2b: shows the measurements of the urinary calprotectin/creatinine concentration ratios in the study population for control subjects, prerenal AKI patients and intrinsic AKI patients after exclusion of patients with urinary tract infections;
- Fig. 3a: shows an ROC curve analysis (discriminatory power) of the urinary calprotectin measurements according to Fig. 1 a;
- Fig. 3b: shows an ROC curve analysis of the calprotectin/creatinine concentration ratio measurements according to Fig. 1b;
- Fig. 3c: shows an ROC curve analysis (discriminatory power) of the urinary calprotectin measurements according to Fig. 2a;
- Fig. 3d: shows an ROC curve analysis of the calprotectin/creatinine concentration ratio measurements according to Fig. 2b;
- Fig. 3e: shows an ROC curve analysis of FE_{Na} measurements for detection of prerenal AKI and intrinsic AKI in the subjects of the same study;
- Fig. 3g: shows an ROC curve analysis of proteinuria measurements for detection of prerenal AKI and intrinsic AKI in the subjects of the same study;
- Fig. 4: shows a diagnosis scheme for the determination of prerenal, postrenal or intrinsic AKI in a patient;
- Fig. 5: is a photograph of a Calprotectin lateral flow tests (Test Format One) with different calprotectin standards for testing the feasibility of a visible threshold for distinguishing between prerenal and intrinsic AKI;
- Fig. 6: is a photograph of a Calprotectin lateral flow tests (Test Format Two) with different calprotectin standards for testing the feasibility of a visible threshold for distinguishing between prerenal and intrinsic AKI;
- Fig. 7: is a column diagram with the readings of the Lateral Flow Reader on Test Format Two Sticks according to the invention.

### DETAILED DESCRIPTION OF THE INVENTION

Calprotectin is a calcium-binding protein produced in neutrophils and monocytes (Bjerke K et al., Gut. 1993; 34(10): 1357-63). The protein is also known as "LI protein", "myeloid related protein 8 - MRP8 - (S100A8) and MRP14 (S100A9)", "cystic fibrosis (associated) antigen (CFA)" and "calgranulin". Calprotectin exists in both dimeric and trimeric forms. As a dimer, calprotectin comprises the polypeptide chains S100A8 and S100A9 (S100A8/S100A9) which are stable in body fluids and stool at room temperature. As a trimer, calprotectin is a 36 kDa heterotrimeric protein with two heavy (14 kD) and one light chain (8 kD) non- covalently linked. In neutrophil cytoplasm it adds up to 60% of the cytosolic proteins (Poullis A, et al., Aliment Pharmacol. Ther, 48, 2002;16(4): 675-81). It is released with so-called "damage-associated molecular pattern proteins (DAMPs)" in a neutrophil granulocyte if stimulated by an invading microorganism.

In gastroenterology, calprotectin in stool is used to differentiate between inflammatory bowel diseases and functional disorders such as irritable bowel syndrome. In inflammatory bowel disease, the migration of stimulated neutrophils into the mucosa leads to highly increased calprotectin concentrations in the stools. Since there is no relevant mucosal inflammation activity in irritable bowel syndrome, fecal calprotectin concentrations are low in this condition. It has been assumed that local calprotectin concentrations are not affected by non-mucosal conditions which otherwise result in an elevation of systemic inflammation markers like C-reactive protein. While calprotectin has previously been determined in urine samples, neither serum nor urinary calprotectin (MRP14) proved suitable to distinguish prostate cancer cases from controls as described by Hermani A et al: Calcium-binding proteins S100A8 and S100A9 as novel diagnostic markers in human prostate Cancer. Clin Cancer Res 2005; 11:5146. To the contrary, urinary calprotectin performed much worse than PSA and was not found useful to reduce false-positive findings of PSA in the controversial range between 4 and 10 ng/ml (Müller H et al., J Urology (2008) 180: 1309-1313, notable 1310, 2"' paragraph, left column). As calprotectin is an inflammation marker, increased serum calprotectin levels have been found to be increased in cases of renal allograft rejection (Burkhardt K et al, J Am Soc Nephrol (2001) 12:1947-57), appendicitis (US 200/0249003 A1), cancers of the urogenital and/or intestinal tract, cardiovascular diseases (EP 1739430 B1), urinary tract infection (UTI) and of course in many digestive diseases.

The present invention is based on the surprising finding that the assessment of the urinary calprotectin level has discriminatory power in cases of acute kidney injury. More precisely, it has been found that urinary calprotectin levels are markedly different in cases of prerenal and intrinsic AKI so that the instant assessment of the urinary calprotectin levels is a most helpful diagnostic tool, in particular, as this can be done by an instant immunoassay such as a lateral-flow immunochromatography. Most importantly, the discriminatory power of the claimed method is not hampered by urogenital tract infections or other inflammations in cases of intrinsic acute kidney injury.

### EXAMPLES

### Example 1

### Urinary calprotectin and ratio of calprotectin/creatinine in patients with AKI

86 patients with AKI of unknown origin and 15 healthy controls (medical staff, no history of hypertension, diabetes, or renal disease) were enrolled in the study. 46 subjects (53.5%) suffered from AKI without any history of renal disease (de novo AKI), 40 subjects (46.5%) had a history of chronic renal failure (acute or chronic renal failure) with a mean creatinine value of 1.4 + 0.8 mg/dl prior to admission. The presence of AKI was defined according to the Acute Kidney Injury Network (AKIN) criteria (Mehta RL et al., Grit. Gare. 2007; 11 (2): R31). Therefore, an increase in serum creatinine of > 0.3 mg/d or an increase to > 1.5 fold from baseline or reduction of diuresis to < 0.5 ml/kg per hour for > 6 hours were defined as inclusion criteria. Each subject was classified as stage 1 to 3 according to AKIN criteria. Patients were excluded for prior renal transplantation and obstructive uropathy.

Diagnoses of prerenal and intrinsic AKI were established as follows: A histological diagnosis of a renal biopsy was used as a standard for a number of patients. In those subjects who did not undergo renal biopsy, the diagnosis was established by predefined clinical criteria (Table 1): Response of renal function to volume repletion was defined as an obligatory criterion (category A) for the diagnosis of prerenal AKI. Compatibility of history, physical findings, and urinary findings were regarded as category B criteria. Diagnosis of prerenal AKI required 2 criteria of category B and the obligatory criterion of category A.

**TABLE I**

| *Clinical Criteria* | *Prerenal AKI* | *Intrinsic AKI* |
|---|---|---|
| A | | |
| Response to volume repletion | Rapid decrease of creatine with convergence to baseline levels | No reconstitution of renal function |

| B | | |
|---|---|---|
| History compatible | Dehydration, loss of fluid, e.g. by gastroenteritis, heart failure, liver failure, inadequate use of diuretics, etc | Prolonged shock, exposition to nephrotoxins, extrarenal suggestive symptoms like pulmorenal syndrome, etc. |
| Physical findings compatible | Low blood pressure, low jugular pulse, tachycardia, orthostatic changes, poor skin turgor) | Absence of signs of dehydration, cardiac monitoring shows adequate volemia |
| Urine examinations | Proteinuria and/or hematuria and/or leukocyturia | Absence of proteinuria, hematuria, and leukocyturia |

Diagnostic criteria of prerenal and intrinsic AKI in the absence of a histological diagnosis. Criteria of category A are obligatory, criteria of category B are optional for the diagnosis of prerenal or intrarenal AKI. Diagnosis of prerenal AKI required > 2 of these criteria including the criterion of category A and absence of all criteria of intrinsic AKI.

The characteristics of the study population are presented in Table II. Intrinsic AKI comprises a multitude of entities including glomerulonephritis, vasculitis, interstitial nephritis, and acute tubular necrosis. Our study population covered all of these causes. The p-value indicates statistical significance.

**TABLE II**

| | Prerenal (n=34) | Intrisic (n=52) | p |
|---|---|---|---|
| Female | 6 (17.6%) | 27 (51.9%) | 0.002 |
| Male | 28 (82.4%) | 25 (48.1%) | |
| Age (years) | 68.4 ± 14.1 | 70.5 ± 16.5 | 0.53 |
| Body mass index (kg/m2) | 26.4 ± 4.9 | 26.1 ± 4.5 | 0.76 |
| Preexisting CKD | 14 (41.2%) | 26 (50%) | 0.51 |
| Biopsy | 1 (2.9%) | 8 (15.4%) | 0.08 |
| Origin of AKI: | | | |
| Dehydration by gastroenteritis | 12 (35.3%) | | |
| Hemodynamic, drug-induced (diuretics, ACE-I, ARB, NSAID) | 11 (32.4%) | | |
| Other causes of dehydration | 5 (14.7%) | | |
| Cardiorenal syndrome | 2 (5.9)% | | |
| Hepatorenal syndrome | 1 (2.9%) | | |
| Bilateral renal artery stenosis | 1 (2.9%) | | |
| Short-term hypotension | 2 (5.9%) | | |
| Hypotension-induced acute tubular necrosis | | 4 (7.7%) | |
| Toxic acute tubular necrosis | | 2 (3.8%) | |
| Crescentic glomerulonephritis | | 3 (5.8%) | |
| Lupus nephritis | | 2 (3.8%) | |
| Other forms of glomerulonephritis | | 4 (7.7%) | |
| Thrombotic thrombocytopenic purpura (TTP) | | 1 (1.9%) | |
| Drug-induced acute interstitial nephritis | | 3 (5.8%) | |
| Renal infarction | | 1 (1.9%) | |
| Rhabdomyolysis | | 1 (1.9%) | |
| Urosepsis/ severe urinary tract infection | | 22 (42.3%) | |
| Contrast-media induced | | 3 (5.8%) | |
| Multiple myeloma | | 1 (1.9%) | |
| Unknown | | 5 (9.6%) | |
| Concomitant diseases: | | | |
| Diabetes mellitus | 10 (29.4%) | 15 (28.8%) | 1.0 |
| Hypertension | 29 (85.3%) | 36 (69.2%) | 0.12 |
| Urinary tract infection | 0 (0%) | 23 (44.2%) | <0.001 |

| Medication on admission: | | | |
|---|---|---|---|
| ACE-1./ARB | 19 (55.9%) | 17 (32.7%) | 0.07 |
| Diuretics | 23 (67.6%) | 22 (42.3%) | 0.04 |
| AKI stage: | | | |
| - stage 1 | 7 (20.6%) | 13 (25.0%) | 0.82 |
| - stage 2 | 9 (26.5%) | 15 (28.8%) | |
| - stage 3 | 18 (52.9%) | 24 (46.2%) | |
| eGFR on admission (ml/min) | 18.2 ± 9.9 | 19.9 ± 13.4 | 0.52 |
| Creatinine on admission (mg/dl) | 4.4 ± 2.7 | 4.1 ± 2.2 | 0.55 |
| Creatinine prior to admission (mg/dl,if available) | 1.4±0.7 | 1.4 ± 0.8 | 0.88 |
| Creatinine at discharge (mg/dl) | 1.7 ± 0.9 | 2.2 ± 1.6 | 0.047 |
| Proteinuria (mg/L) | 97.5 (65.8 - 177.5) | 393.0 (167.0- 1023.0) | <0.001 |
| Proteinurie (mg/L)/urinary creatinine (g/L) | 164.9 (74.5 - 308.7) | 986.7 (295.3 - 2071.0) | <0.001 |
| C-reactive protein (mg/dl) | 5.2 ± 4.6 | 6.7 ± 9.7 | 0.40 |
| Urinary creatinine (g/L) | 0.71 ± 0.32 | 0.61 ± 0.38 | 0.23 |
| Urinary sodium (mmol/L) | 67.0 ± 32.4 | 67.5 ± 39.4 | 0.95 |
| Plasma sodium (mmol/L) | 137.6 ± 4.5 | 137.4 ± 5.5 | 0.90 |
| Fractional excretion of sodium (%) | 3.5 ± 2.5 | 5.2 ± 6.5 | 0.10 |
| Urinary calprotectin (ng/ml) | 27.9 (12.5 - 73.2) | 1692.2 (765.4 - 4734.6) | <0.001 |
| Urinary calprotectin (ng/ml)/urinary creatinine (g/L)ratio | 52.0 (23.3 - 123.4) | 3697.7 (1329.8 - 7812.3) | <0.001 |

Characteristics of study population; Numeric data are presented as mean ± standard deviation in case of normal distribution, otherwise as median and interquartile range (calprotectin, calprotectin/creatinine ratio, proteinuria). P < 0.05 was regarded statistically significant.

Urine concentrations of calprotectin were quantified using a commercial enzyme-linked immunosorbent assay (ELISA) kit (PhiCal® Calprotectin, Immundiagnostik AG, Bensheim, Germany) according to the manufacturer's protocol in a blinded fashion. Each 100-1 sample of diluted urine (1:10) or standard was placed in a microtiter plate coated with a monoclonal antibody against human calprotectin heterodimer. After incubation for 1 h at 37°C on a horizontal mixer the wells were washed 5 times with buffer, before a biotinylated secondary monoclonal anti-human calprotectin antibody was added to each microtiter well (100 l), After washing the well again, a peroxidise-labelled streptavidin conjugate was added. After incubation at room temperature for 1 h, excess conjugate was removed by washing. Tetramethylbenzidine solution (100 l) was used as a substrate for peroxidase (20 min incubation at darkness, room temperature). Finally, sulfuric acid solution (100 l) was added to terminate the reaction. Absorbances were measured at 450 nm against 620 nm as reference. The coefficient of variation for duplicate measurements using this ELISA kit was < 6 %.

In order to take the current concentration status of the urine into account, creatinine was assessed in the urine and calprotectin/creatinine relations were calculated.

The subjects taking part in the study were classified according to biopsy results or- if not available- to the criteria presented in Table I. This revealed 34 cases of prerenal AKI and 52 cases of intrinsic AKI. Healthy controls (9 male, 6 female) had a mean age of 33.8 ± 10.0 years and a BMI of 23.4 ± 3.6 kg/m². Serum creatinine was 0.86 ± 0.12 mg/dl corresponding to an eGFR of 96.4 ± 22.2 ml/min. The epidemiological data, stage and origin of AKI, concomitant diseases, premedication, renal parameters, and findings of calprotectin measurements in the patients with AKI are presented in Table II (above). No significant difference between the prerenal and intrinsic AKI group was observed concerning age, BMI, preexisting chronic kidney disease, diabetes, and hypertension (p > 0.05 each). Performance of biopsy tended to be more frequent in intrinsic AKI (p = 0.08). Premedication with ACE-inhibitors or angiotensin receptor 5 blockers tended to be more frequent in prerenal disease (p = 0.07), intake of diuretics was significantly more frequent in prerenal disease (p = 0.04). Compared to intrinsic AKI, male gender was predominant in prerenal disease (p = 0.002).

### Results

The most frequent cause of prerenal AKI was dehydration due to gastroenteritis followed by drug-induced hemodynamic AKI (diuretics, ACE-inhibitors, angiotensin receptor blockers, non-steroidal anti-inflammatory drugs). These two entities constituted two thirds of all cases of prerenal AKI (Table II). The remaining third was caused by other causes of dehydration, short-term hypotension, hepatorenal syndrome, cardiorenal syndrome and bilateral renal artery stenosis. The most frequent cause of intrinsic AKI was severe urinary tract infection, followed by glomerulonephritis, acute tubular necrosis, contrast media induced renal failure, interstitial nephritis, renal infarction, thrombotic thrombocytopenic purpura (TTP), rhabdomyolysis, and multiple myeloma (Table II). In both prerenal and intrinsic AKI, the majority of cases corresponded to stage 3 according to AKIN-criteria followed by stage 2 and stage 1. Severity of AKI as measured by AKIN-criteria was not significantly different between the prerenal and intrinsic AKI group (p = 0.82). Accordingly, creatinine and eGFR on admission were not significantly different (p = 0.55, p = 0.52). Creatinine at discharge from hospital, however, was significantly lower in prerenal AKI (p = 0.047) and values tended to baseline levels measured prior to admission. The two groups differed significantly in the amount of proteinuria being 6 times higher in intrinsic AKI (p < 0.001). C-reactive protein, urinary creatinine, urinary sodium and plasma sodium were not significantly different in the two groups (p > 0.05 each). FE_{Na} tended to be higher in intrinsic AKI but the difference did not reach any significance (p = 0.10).

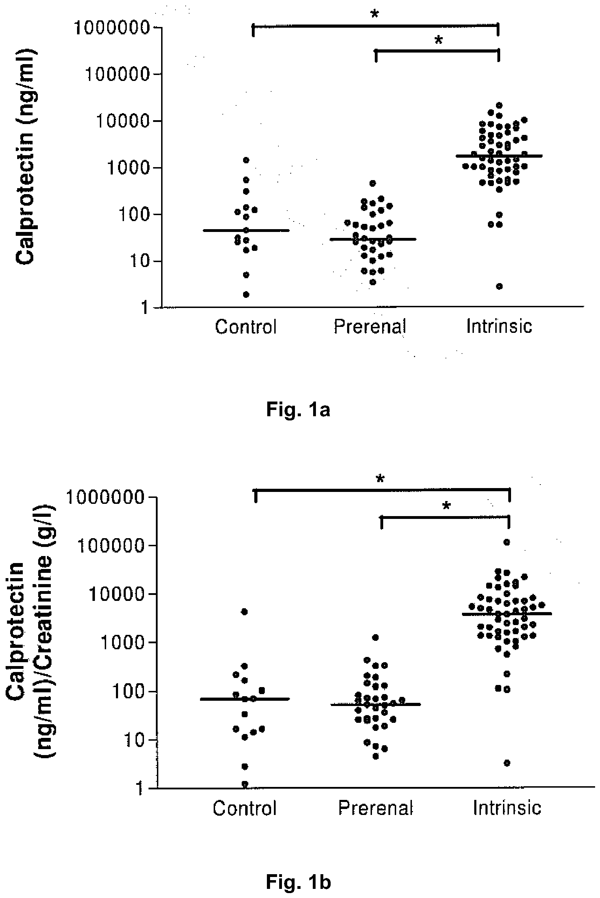

Assessment of urinary calprotectin concentration was successful in the whole study population. Figs. 1a and 1b present the individual results of calprotectin measurements and concentration ratio calprotectin/creatinine measurements in each group respectively. Values for calprotectin ranged from 0-20400 ng/ml. In order to take the current concentration status of the urine into account, urinary creatinine was assessed and calprotectin/creatinine ratios were calculated ranging from 0 to 112710 (ng/ml) / (g/L). Median calprotectin concentrations were not significantly different in healthy controls (44 8 ng/ml, interquartile range 18.7 to 138.9) and prerenal AKI (27.9 ng/ml, interquartile range 12.5 to 73.2, p = 0.25). Median urinary calprotectin was 60.7 times higher, however, in intrinsic AKI (1692.2 ng/ml, interquartile range 765.4 to 4734.6) than in prerenal AKI (p<0.001).

Calprotectin/creatinine ratios did not differ significantly between healthy controls (69.5 (ng/ml)/ (g/l), interquartile range 14.2 to 162.3) and prerenal AKI (52.0 (nglml)/ (g/l), interquartile range 23.3 to 123.4, p=0.97). The calprotectin/creatinine ratio in intrinsic AKI, however, was 71.1 times higher than in prerenal AKI (p < 0.001). The calprotectin and calprotectin/creatinine ratios in intrinsic AKI were significantly higher than those of healthy controls (p < 0.001 each).

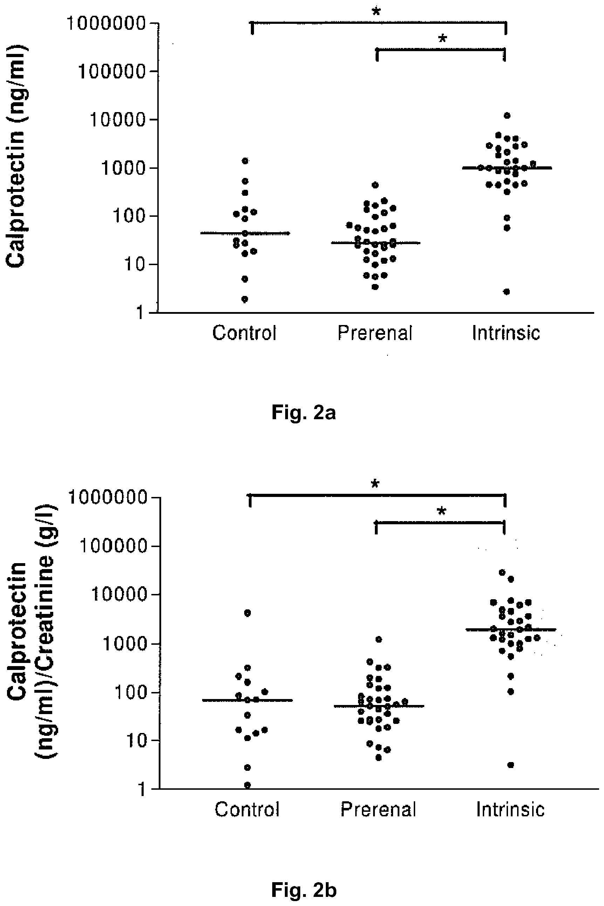

Since urinary tract infections go along with leukocyturia potentially leading to high urinary calprotectin concentrations, all cases of urinary tract infection were excluded in a second analysis. Figs. 2a and 2b illustrate the results of this analysis showing that exclusion of urinary tract infection did not lead to a qualitative change of the findings in the overall study population. There were no cases of urinary tract inlection in the prerenal AKI group; healthy controls were not tested for urinary tract infection. Accordingly, calprotectin and calprotectin/creatinine ratios did not change in these groups. Urinary calprotectin concentration was 36.1 times higher in intrinsic AKI (1007.1 ng/ml, interquartile range 465.0 to 2675.4) than in prerenal AKI (p < 0.001). The calprotectin/creatinine ratio was 38.2 times higher in intrinsic AKI (1987.4 (ng/ml)/ (g/l), interquartile range 1008.6 to 4744.0) when compared to prerenal AKI (p < 0.001).

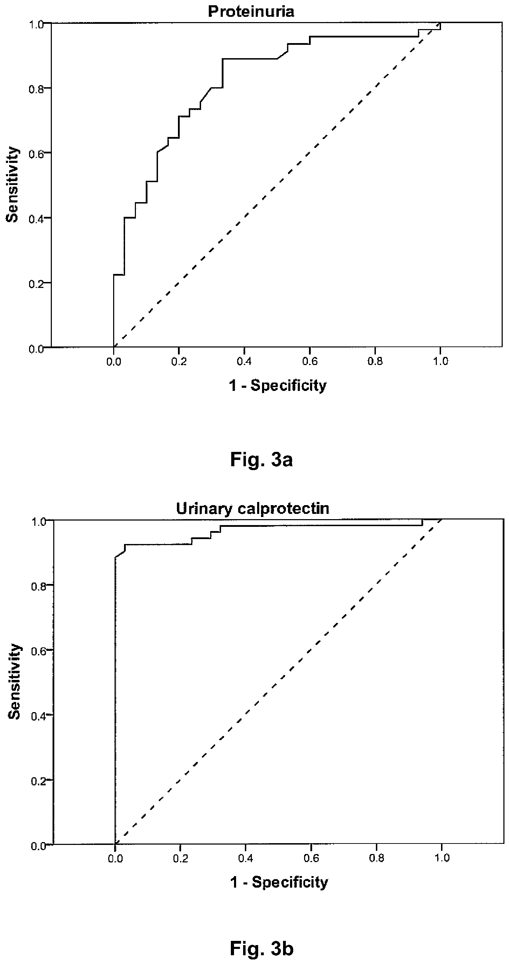

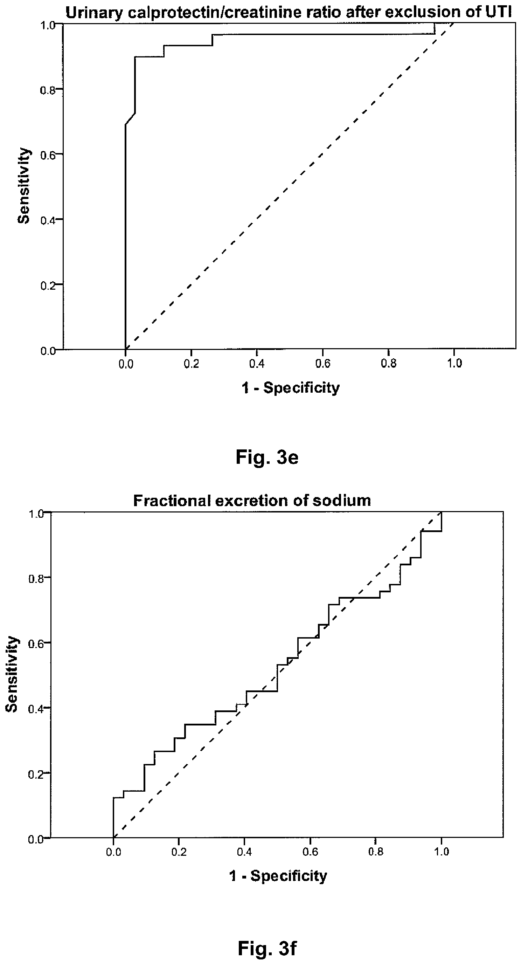

The accuracy of urinary calprotectin in the detection of intrinsic AKI was assessed by ROC curve analysis as presented in Figs. 3a to 3f. Receiver-operating characteristic curve (ROC) analysis of calprotectin revealed an area under the curve (AUC) of 0.965 (Fig. 3a), the calprotectin/creatinine ratio had an AUC of 0.966 (Fig. 3b). FE_{Na} performed worse with an AUC of 0.524 (Fig. 3e). Proteinuria (AUC 0.819) was more accurate than FE_{Na} but worse than calprotectin and the calprotectin/creatinine ratio (Fig. 31). ROC analysis was used to determine the optimal cut off value for the differentiation of prerenal and intrinsic AKI.

**TABLE III**

| | Overall study population | | Subjects without urinary tract infection | |
|---|---|---|---|---|
| | Calprotectin (ng/mL) (Cut off 300) | Calprotectin (ng/mL) / creatinine (g/L) (Cut off 500) | Calprotectin (ng/mL) (Cut off 300) | Calprotectin (ng/mL) / creatinine (g/L) (Cut off 500) |
| Sensitivity (%) | 92.3% | 92.3% | 89.7% | 89.7% |
| Specificity (%) | 97.1% | 97.1% | 97.1% | 97.1% |
| Positive predictive value (%) | 98.0% | 98.0% | 96.3% | 96.3% |
| Negative predictive value (%) | 89.2% | 89.2% | 91.7% | 91.7% |

Performance of urinary calprotectin, calprotectin/creatinine ratio, and fractional excretion of sodium for the detection of intrarenal AKI in the overall study population and after exclusion of urinary tract infection.

As presented in Table III, calprotectin achieved a sensitivity of 92.3% and a specificity of 97.1% for a threshold value of 300 ng/ml. The resulting positive and negative predictive values were 98.0% and 89.2%, respectively. Calprotectin/creatinine ratio achieved the same accuracy parameters with a cut off value of 500 ng/ml calprotectin to g/mL reatinine. This corresponds to an absolute concentration by weight ratio of 0.0005 calprotectin/creatinine.

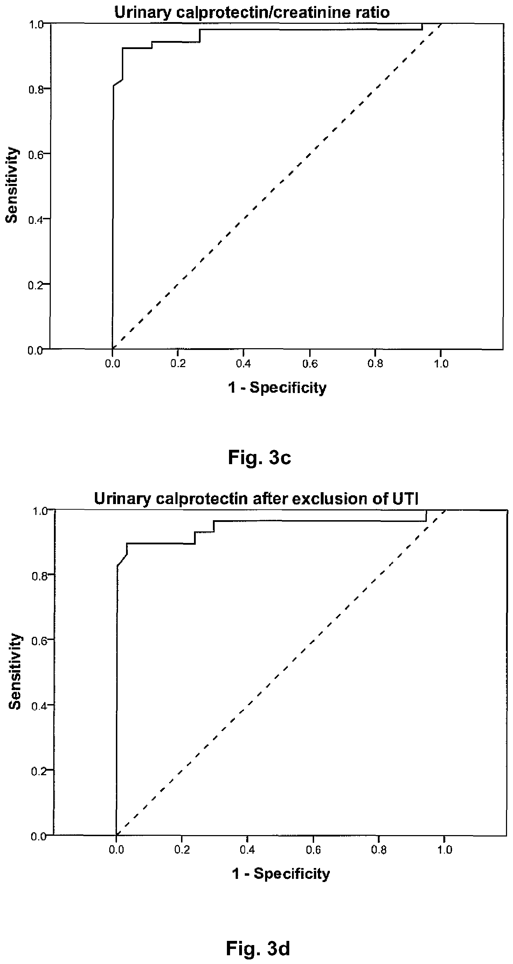

After exclusion of all cases with urinary tract infection, the ROC curve's AUC was 0.948 for calprotectin (Fig. 3c) and 0.949 for the calprotectin/creatinine ratio (Fig. 3d). Specificity remained 97.1%, and sensitivity decreased slightly to 89.7% for both parameters. Accordingly, the positive predictive value decreased slightly to 96.3%, whereas the negative predictive value increased to 91.7%. The achieved accuracy of urinary calprotectin in diagnosing intrinsic AKI is much higher than that of current diagnostic markers like FE_{Na} or proteinuria. The relatively low accuracy of FE_{Na} may be explained primarily by the high percentage of patients with diuretics (43.7%) leading to high natriuresis even in prerenal AKI. A sensitivity of FE_{Na} of 78% in patients not administered with diuretics and 58% in those administered with diuretics is known (Pepin MN et al.: Am J Kidney Dis. 2007; 50 (4), 566-573).

The high percentage of patients with diuretic therapy in the present study population adequately reflects the situation in today's clinical practice, since the incidence of AKI increases with age and the presence of congestive heart failure. Since proteinuria is not affected by diuretic therapy, this marker was more accurate in diagnosing intrinsic AKI in our study population.

Despite its many limitations, FE_{Na} is still being regarded as the gold standard laboratory marker in the differential diagnosis of AKI. The accuracy of calprotectin with a sensitivity and specificity of 92.3% and 97.1% is much better than FE_{Na}. Urinary calprotectin has proven to be the most accurate diagnostic parameter in the differential diagnosis of AKI.

### Discussion of results

Calprotectin is mainly derived from neutrophil granulocytes. Urinary tract infection largely increases urinary calprotectin, since it goes along with leukocyturia. Thus, urinary tract infections with or without coincidental signs of dehydration - as being a reason of deteriorating renal function - would be classified as intrarenal AKI. A second accuracy analysis after exclusion of all subjects with urinary tract infection was therefore performed. Interestingly, the ROC AUC as a measure of diagnostic accuracy of urinary calprotectin decreased only minimally from 0.965 to 0.948. The positive predictive value decreased slightly from 98 to 96.3%, whereas the negative predictive value even increased from 89.2 to 91.7%. Hence, urinary tract infections do not significantly influence the test results.

In the healthy control group, three subjects with no history of renal disease had calprotectin concentrations of > 300 ng/ml including one person with a concentration of even 1413 ng/ml. All of these persons were female. All of the male probands had urinary calprotectin levels of <150 ng/ml. The two most evident reasons are the higher percentage of asymptomatic bacteriuria in females and potential (peri)- menstrual blood contaminations of the urine. It was concluded that low urinary calprotectin concentration largely excludes a severe intrarenal damage in both genders. In case of a high calprotectin concentration in females, menstrual bleeding and urinary tract infection should be ruled out.

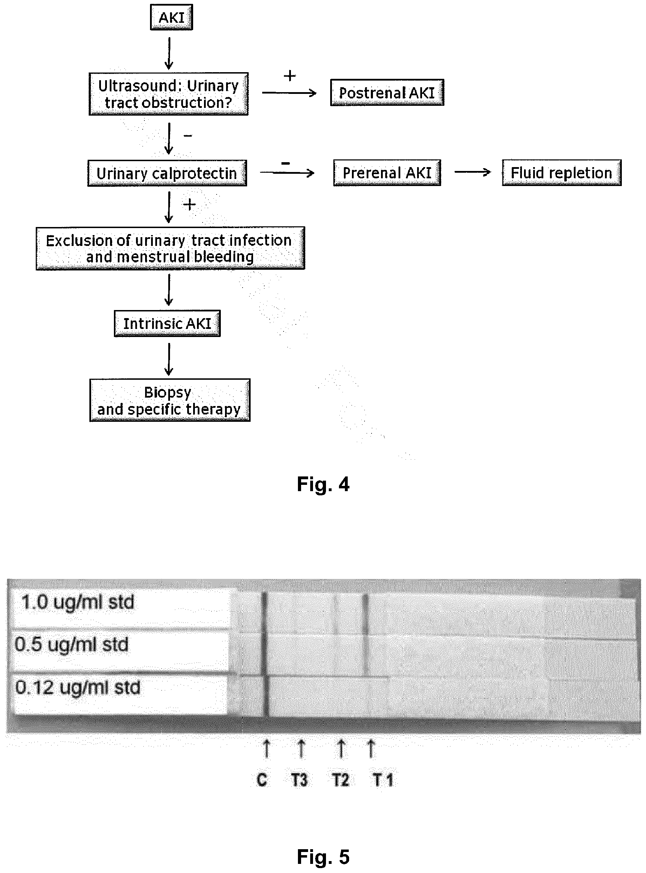

It was found that urinary calprotectin and the urinary concentration ratio calprotectin/creatinine are reliable indicators for intrinsic AKI and their detection provides good non-invasive methods for diagnosing intrinsic AKI and for differentiation between intrinsic and prerenal AKI. These findings have lead to a diagnostic scheme as shown in Fig. 4 for determination of prerenal, postrenal or intrinsic AKI and for deciding on patient treatment of AKI in a patient.

### Example 2

### Lateral-flow immunochromatography

As a representative example, a kit for the detection of calprotectin in urine was developed. The development included the steps (i) coupling and labelling of anti-calprotectin antibodies with biotin and digoxigenin; (iii) testing of the obtained antibodies on standard biotin-digoxigenin test strip quick tests with different samples; (iv) adaptation and calibration of the calprotectin internal standard to the required detection limit of the test strip.

i) *Coupling of capture and detection antibodies against calprotectin with digoxigenin and biotin.* A first monoclonal antibody (K6930A2 of Immundiagnostik AG, Bensheim, OE) was labelled with digoxigenin and a second polyclonal (polyclonal rabbit anti-calprotectin) with biotin. Digoxigenation was carried out using a digoxigenin-labelling kit of Roche Diagnostik GmbH, Mannheim (DIG-Protein Labelling Kit Kat Nr. 11 367 200 001). In brief, digoxigenin-3-0-succinyl- -aminocapronic acid N-hydroxy-succinimide ester (DIG-NHS) was dissolved in 50 µL DMSO and added to the antibody solution (1 mL) in a molar ratio of 5:1 (1 antibody molecule per 5 molecules DIG-NHS). The reaction was stopped by addition of L-lysine, and the antibodies were separated by fractionation on a Sephadex-G-25 and dialysis of excess labelling reagent.

Biotinylation was carried out using a biotin labelling kit of Roche Diagnostik GmbH, Mannheim (Biotin Protein Labelling Kit Kat Nr.11 418165 001). In particular, D-biotinyl- -aminocapronic acid N-hydroxysuccinimide ester (biotin-7-NHS) was dissolved in DMSO and added to the antibody solution (1 ml) in a molar ratio of 5:1 (1 antibody molecule per 5 molecules biotin-7-NHS; 2 hours at room temperature). The reaction was stopped by addition of L-lysine, and the antibodies were separated by fractionation on a Sephadex-G-25, followed by a dialysis of excess labelling reagent. The digoxigenated and biotinylated antibodies were then set to 1 mg/ml PBS, 0.2 % sodium azide and frozen.

(ii) *Thin-layer test strips for immune chromatography.* Anti-biotin/anti-digoxigenin quick test strips of Roche Diagnostik GmbH, Mannheim were used. On the test strip, the digoxigenin-reporter molecule is dyed with gold-labelled anti-digoxigenin antibodies in the impregnated zone of the quick-test strip. The dyed sandwich complex may then be detected in the immuno-thin layer chromatography through its binding to streptavidin, as described above.

(iii) *Testing of the biotinylated and digoxigenated anti-calprotectin-antibodies on standard biotin-digoxigenin quick test strips.* 400 µL urine sample was transferred into a reaction vessel and 2.5 µL each of biotinylated and digoxigenated anti-calprotectin antibodies added to each. The sample was mixed and left standing for 10 minutes for the formation of the sandwich complex. Afterwards, quick test strips were positioned into the solution and the result read off after 4 minutes.

In the following step the lateral-flow assay has to be adapted and calibrated to the required threshold concentration (300 ng /ml).

### Example 3

*Feasibility study to demonstrate if a Calprotectin Lateral Flow Test could be used to visually distinguish between 120, 500 and 1000 ng*/*mL samples.*

Two potential test formals have been investigated to meet the requirements. Both are currently based on a system with a first anti-CP (monoclonal mouse) antibody conjugated to 40 nm gold-colloid and a second anti-CP antibody used as the capture reagent. Format one consists of three anti-calprotectin test lines of graduated concentration on a single stick. Format two consists of three separate sticks, each with a different anti-Calprotectin test line concentration. Both showed a good visual differential between 0.12, 0.5 and 1.0 ug/ml calprotectin standard samples. Furthermore, both can be read by the Lateral Flow Reader and produced numerical evidence of this differential. Using the three lines, either on single or multiple sticks, it is proposed that a number of different test line profiles can be produced, allowing the physician to distinguish between varying degrees of calprotectin positivity.

**TABLE IV**

| MATERIAL | DESCRIPTION | SUPPLIER | PART NUMBER |
|---|---|---|---|
| Anti-CP antibody | 4mg/ml lot LI860 | Immundiagnostik, Bensheim | K99991AK |
| Calprotectin | 140 ug/ml lot 10/03/04 | Immundiagnostik, Bensheim | N/A |
| Running Buffer | PBS | Immundiagnostik AG | N/A |
| Nitrocellulose | CNPFIOu, 20 mm, | MDI | R-5535 |
| Colloidal Gold | 40 nm | Alchemy Laboratories, UK | N/A |
| Control line solution | Anti-mouse Ab 0.5 mg/ml | Alchemy Laboratories, UK | N/A |
| Treated Release Pad | Conjugate Pad 2, 19 mm | Alchemy Laboratories, UK | N/A |
| Sample Pad | 15 mm, untreated | Alhstrom | 1281 |
| Absorbent Pad | 27 mm | Alhstrom | 222 |

*Test Format One, Three Line:* three separate test lines were coated on the same strip of membrane, at three different concentrations. Control line antibody was also coated on the membrane, giving four lines in total.

*Test Format Two, Three sticks:* separate strips of membrane were coated with three different test line concentrations. Control line antibody was coated on the membrane, giving two lines on each membrane.

Both types of card were coated and dried following standard procedures. Gold conjugate was prepared with K999AK antibody and conjugation conditions were optimised. Coated cards were assembled with specifically treated release pad, sample pad and absorbent pad. Assembled cards were cut to 5 mm. Gold conjugate was coated onto release pad and strips were then dried at 37°C.

Standards were prepared at 1.0, 0.5, 0.12 and 0.06 g/ml in PBS buffer. 100 ul of each standard was run with the strips, as well as PBS only. After 15 minutes, a photograph of the test strips was taken. Strips from Test Format Two were also read on the Lateral Flow Reader. The results for the Test Format One are shown in Figure 5 and Table V and VI.

**TABLE V**

| *Signal Profiles* of *Test Format One - Three Line: With three lines of different capture antibody concentration and fixed qty of gold conjugate* | | | |
|---|---|---|---|
| STANDARD CONC. (µg/mL) | TEST LINE 1 | TEST LINE 2 | TEST LINE 5 |
| 1.0 | +++ | ++ | + |
| 0.5 | ++ | + | Trace |
| 0.12 | + | Trace | |

**TABLE VI**

| *Signal Profiles* of *Test Format Two - Three sticks coated with lines of different capture antibody concentration and fixed qty of gold conjugate* | | | |
|---|---|---|---|
| STANDARD CONC. (µg/mL) | TEST LINE 1 | TEST LINE 2 | TEST LINE 5 |
| 1.0 | +++ | ++ | + |
| 0.5 | ++ | ++ | Trace |
| 0.12 | + | Trace | - |

The above results demonstrate that each of the standard concentrations can feasibly be distinguished visually according to a unique test line signal profile. This finding is applicable to both test Formats One and Two, although further work would need to be completed to optimize both these systems and they have only been conducted with buffer standards.

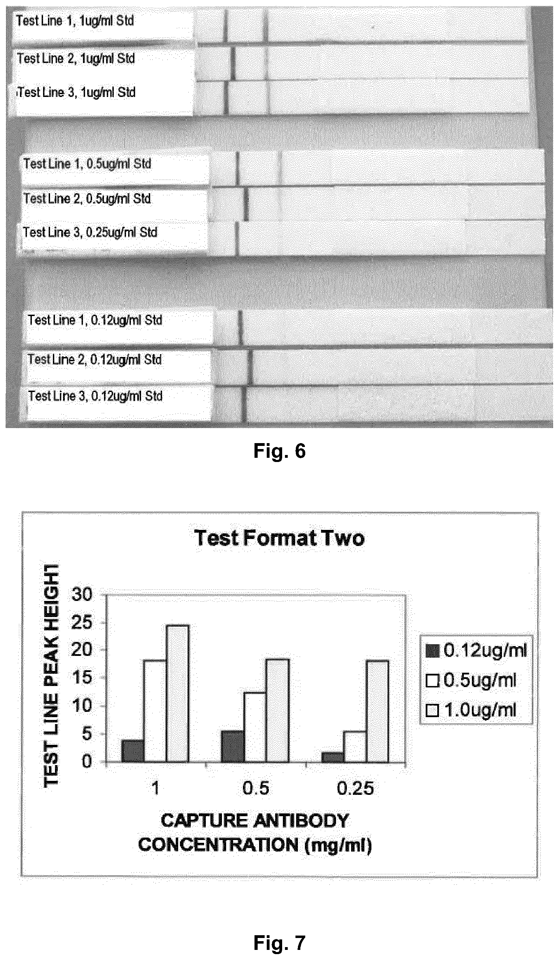

Test Format Two sticks were also read using the Lateral Flow Reader. The results are shown in Figure 7. These results reinforce the signal profiles generated visually and show a test line peak height reduction with decreasing capture antibody concentration. This is particularly obvious with respect to the 1.0 and 0.5 µg/mL standards. The 0.12 µg/mL standard does show some variability, however this is not significant enough to interfere with the overall profile produced for each standard, and hence does not alter test interpretation.

Using the reader a semi quantifiable test is possible, based on the current buffer system and standards. A fully quantifiable test may be possible with a reader and optimized buffer systems.

Visually, differential between standards was possible with both three-test line and three individual tests. For totally definitive cut offs at each level, the use of a comparator or reference line is required in the present system.

### Example 4

### Urinary calprotectin concentrations and renal allograft injury

Urine samples of 31 renal allograft recipients (7 live donors and 24 deceased donors; 16 male and 15 female; age 54, range 47-65 years) were collected at day one to five after transplantation. 13 healthy probands served as control. Urinary calprotectin concentrations were measured by ELISA (PhiCal®), Immundiagnostik AG, Bensheim, Germany). All data below are presented as median and range (2nd to 4th quartile).

Urinary calprotectin concentrations were higher after transplantation than in healthy controls (1911 ng/ml, range 484-4180 ng/ml vs. 45 ng/ml, range 18-131 ng/ml, p<0.001). Calprotectin was significantly lower after live donor than after deceased donor transplantation (484 ng/ml, range 178-1481 ng/ml vs. 2050 ng/ml, range 604-7849 ng/ml; p 0.03) and in those patients with primary graft function (n=23; 1482 ng/ml, range 352-3357 ng/ml) compared to delayed graft function (8767 ng/ml, range 1911-9381 ng/ml; p 0.029). Cold ischemia time was significantly lower in those patients with a urinary calprotectin below the median compared to those above the median (290 min, range 175-811 min vs. 626 min, range 445-880 min; p 0.045).

Urinary calprotectin concentrations early after renal transplantation reflect allograft injury, e.g. induced by ischemia. Thus, calprotectin (S100A8/S100A9) is also a urinary biomarker of acute kidney injury (AKI) in renal allograft recipients. A consequence of the kidney ischemia and reperfusion is capillary damage and a damage of tubuli which corresponds pathomorphologically to an acute kidney injury (AKI). Thus, the initial urinary calprotectin concentrations reflect the damage and repair processes of the tubuli alter renal ischemia and initial high urinary calprotectin concentrations correspond to an AKI alter renal ischemia. Our data suggest !hat urinary calprotectin concentrations should be back to normal levels about six months alter renal transplantation. A renewed increase of urinary calprotectin concentrations would then support the diagnosis of an acute kidney injury due to a failure of the graft.

## Claims

1. Method of diagnosis of intrinsic acute kidney injury comprising the steps of
**i)** taking a urine sample from a patient suspected of suffering from intrinsic acute kidney injury;
**ii)** assessing the level of calprotectin in the urine sample obtained in step (i);
**iii)** relating the urinary calprotectin level determined in step (ii) to a pre-selected threshold level,
wherein a urinary calprotectin level higher than said pre-selected threshold level indicates that the patient is suffering from intrinsic acute kidney injury.

2. Method of diagnosis of prerenal acute kidney injury comprising the steps of
**i)** taking a urine sample from a patient suspected of suffering from prerenal acute kidney injury;
**ii)** assessing the level of calprotectin in the urine sample obtained in step (i);
**iii)** relating the urinary calprotectin level determined in step (ii) to a pre-selected threshold level,
wherein a urinary calprotectin level lower than said pre-selected threshold level indicates that the patient is suffering from prerenal acute kidney injury.

3. Method of diagnosis as claimed in claim 1 or claim 2, further comprising the step of determining the level of creatinine in the urine sample obtained in step (i) and calculating the weight ratio of urinary calprotectin to creatinine.

4. Method of diagnosis according to claim 1 or claim 2, wherein said threshold level is pre-selected to provide a diagnosis of intrinsic or prerenal acute kidney injury having a sensitivity of higher than 90% or a specificity of higher than 90% or both.

5. Method of diagnosis according to any claim 1 to 4, wherein said pre-selected threshold level corresponds to approximately 300 ng calprotectin per milliliter urine sample or a weight ratio of 0.0005 for urinary calprotectin to creatinine.

6. Method according to any claim 1 to 5, wherein the urinary calprotectin level is assessed by lateral flow immunochromatography.

7. Method according to any claim 1 to 6, wherein the urinary calprotectin level is assessed by an enzyme-linked immuno-sorbent assay.

8. Use of a test kit in a method of assessing the urinary calprotectin level in a urine sample of a patient, wherein said test kit comprises a lateral-flow immunochromatographic device wherein a calprotectin-receptor complex is formed, if calprotectin is present in the sample, and which is calibrated to produce a visible coloring when the urinary calprotectin level corresponds to at least approximately 300 ng calprotectin per milliliter urine sample.

9. Use of a test kit according to claim 8, wherein the test kit further comprises a vessel with a known amount of calprotectin to provide a visible reference coloring.

10. Use of a test kit, wherein said test kit comprises a lateral-flow immunochromatographic device wherein a calprotectin-receptor complex is formed, for instant assessment of the urinary calprotectin level in a method for differentiating between intrinsic and prerenal acute kidney injury.

11. Method as claimed in any claim 1 to 7, and if said patient is diagnosed with intrinsic acute kidney injury, examining a renal biopsy which has been obtained from said patient for further determining the nature of the acute kidney injury and determining an appropriate therapy.

## Patentansprüche

1. Verfahren zur Diagnose eines intrinsischen akuten Nierenschadens, umfassend die Schritte:
**i)** Entnahme einer Urinprobe von einem Patienten, von welchem vermutet wird, dass er an einem intrinsischen akuten Nierenschaden leidet,
**ii)** Bestimmung eines Calprotectin-Spiegels in der in Schritt (i) entnommenen Urinprobe,
**iii)** Vergleich des Urin-Calprotectin-Spiegels, welcher in Schritt (ii) bestimmt wurde, mit einem vorher bestimmten Schwellenwert,
wobei ein Urin-Calprotectin-Spiegel, welcher höher als besagter vorher bestimmter Schwellenwert ist, anzeigt, dass der Patient an einem intrinsischen akuten Nierenschaden leidet.

2. Verfahren zur Diagnose eines prärenalen akuten Nierenschadens, umfassend die Schritte:
**i)** Entnahme einer Urinprobe von einem Patienten, von welchem vermutet wird, dass er an einem prärenalen akuten Nierenschaden leidet,
**ii)** Bestimmung eines Calprotectin-Spiegels in der in Schritt (i) entnommenen Urinprobe,
**iii)** Vergleich des Urin-Calprotectin-Spiegels, welcher in Schritt (ii) bestimmt wurde, mit einem vorher bestimmten Schwellenwert,
wobei ein Urin-Calprotectin-Spiegel, welcher niedriger als besagter vorher bestimmter Schwellenwert ist, anzeigt, dass der Patient an einem prärenalen akuten Nierenschaden leidet.

3. Verfahren zur Diagnose wie beansprucht in Anspruch 1 oder 2, weiterhin umfassend den Schritt, dass der Kreatininspiegel in der in Schritt (i) entnommenen Urinprobe bestimmt wird, und dass das Gewichtsverhältnis von Calprotectin zu Kreatinin im Urin bestimmt wird.

4. Verfahren zur Diagnose wie beansprucht in Anspruch 1 oder 2, wobei besagter Schwellenwert so gewählt ist, dass die damit ermöglichte Diagnose eines intrinsischen oder prärenalen akuten Nierenschadens eine Sensitivität von mehr als 90 %, oder eine Spezifität von mehr als 90 %, oder beides, aufweist.

5. Verfahren zur Diagnose wie beansprucht in Anspruch 1 bis 4, wobei besagter Schwellenwert ungefähr 300 ng Calprotectin pro Milliliter Urinprobe, oder einem Gewichtsverhältnis von Calprotectin zu Kreatinin von 0.0005 entspricht.

6. Verfahren gemäß einem der Ansprüche 1 bis 5, wobei der Urin-Calprotectin-Spiegels durch Lateral Flow Immunochromatographie bestimmt wird.

7. Verfahren gemäß einem der Ansprüche 1 bis 6, wobei der Urin-Calprotectin-Spiegels durch Enzym-gekoppelten Immunoadsorptionsassay (ELISA) bestimmt wird.

8. Gebrauch eines Testkits in einem Verfahren zur Bestimmung des Urin-Calprotectin-Spiegels in einer Urinprobe eines Patienten, wobei besagtes Testkit eine Vorrichtung zur Lateral Flow Immunochromatographie umfasst, in welcher ein Calprotectin-Rezeptor-Komplex gebildet wird, wenn Calprotectin in der Probe vorhanden ist, und welche so kalibriert ist, dass eine sichtbare Färbung hervorgerufen wird, wenn der Urin-Calprotectin-Spiegel mindestens ungefähr 300 ng Calprotectin pro Milliliter Urinprobe entspricht.

9. Verwendung eines Testkits gemäß Anspruch 8, wobei das Testkit weiterhin ein Gefäß mit einer bekannten Menge an Calprotectin umfasst, um eine sichtbare Referenzfärbung zu ermöglichen.

10. Verwendung eines Testkits, wobei besagtes Testkit eine Vorrichtung zur Lateral Flow Immunochromatographie umfasst, in welcher ein Calprotectin-Rezeptor-Komplex gebildet wird, zur sofortigen Bestimmung des Urin-Calprotectin-Spiegels in einem Verfahren zur Differenzierung zwischen intrinsischem und prärenalen akuten Nierenschaden.

11. Verfahren wie beansprucht in einem der Ansprüche 1 bis 7, und wenn bei besagtem Patienten intrinsischer akuter Nierenschaden diagnostiziert wird, untersuchen einer Nierenbiopsie, welche dem Patienten entnommen wurde, zur weiteren Bestimmung der Natur des akuten Nierenschadens und zur Bestimmung einer angemessenen Therapie.

## Revendications

1. Méthode de diagnostic d'une insuffisance rénale aiguë intrinsèque comprenant les étapes consistant à
i) prélever un échantillon d'urine d'un patient soupçonné de souffrir d'une insuffisance rénale aiguë intrinsèque ;
ii) évaluer le taux de calprotectine dans l'échantillon d'urine obtenu à l'étape (i) ;
iii) mettre en relation le taux calprotectine urinaire déterminé à l'étape (ii) avec un taux seuil présélectionné,
dans laquelle un taux calprotectine urinaire supérieur audit taux seuil présélectionné indique que le patient souffre d'insuffisance rénale aiguë intrinsèque.

2. Méthode de diagnostic d'une insuffisance rénale aiguë prérénale comprenant les étapes consistant à
i) prélever un échantillon d'urine d'un patient soupçonné de souffrir d'une insuffisance rénale aiguë prérénale ;
ii) évaluer le taux de calprotectine dans l'échantillon d'urine obtenu à l'étape (i) ;
iii) mettre en relation le taux calprotectine urinaire déterminé à l'étape (ii) avec un taux seuil présélectionné,
dans laquelle un taux calprotectine urinaire inférieur audit taux seuil présélectionné indique que le patient souffre d'insuffisance rénale aiguë prérénale.

3. Méthode de diagnostic selon la revendication 1 ou la revendication 2, comprenant en outre l'étape consistant à déterminer le taux de créatinine dans l'échantillon d'urine obtenu à l'étape (i) et à calculer le rapport pondéral de la calprotectine à la créatinine dans l'urine.

4. Méthode de diagnostic selon la revendication 1 ou la revendication 2, dans laquelle ledit taux seuil est présélectionné pour fournir un diagnostic d'insuffisance rénale aiguë intrinsèque ou prérénale ayant une sensibilité supérieure à 90 % ou une spécificité supérieure à 90 %, ou l'une et l'autre.

5. Méthode de diagnostic selon l'une quelconque des revendications 1 à 4, dans laquelle ledit taux seuil présélectionné correspond à approximativement 300 ng de calprotectine par millilitre d'échantillon d'urine ou un rapport pondéral de 0,0005 pour le rapport de la calprotectine à la créatinine dans l'urine.

6. Méthode selon l'une quelconque des revendications 1 à 5, dans laquelle le taux calprotectine urinaire est évalué par immunochromatographie sur membrane.

7. Méthode selon l'une quelconque des revendications 1 à 6, dans laquelle le taux calprotectine urinaire est évalué par méthode enzymo-immunologique ELISA.

8. Utilisation d'une trousse de test dans une méthode d'évaluation du taux calprotectine urinaire dans un échantillon d'urine d'un patient, dans laquelle ladite trousse de test comprend un dispositif d'immunochromatographie sur membrane dans laquelle un complexe calprotectine-récepteur est formé, si de la calprotectine est présente dans l'échantillon, et qui est étalonnée pour produire une coloration visible lorsque le taux calprotectine urinaire correspond à au moins approximativement 300 ng de calprotectine par millilitre d'échantillon d'urine.

9. Utilisation d'une trousse de test selon la revendication 8, dans laquelle la trousse de test comprend en outre un récipient avec une quantité connue de calprotectine pour fournir une coloration de référence visible.

10. Utilisation d'une trousse de test, dans laquelle ladite trousse de test comprend un dispositif d'immunochromatographie sur membrane dans laquelle un complexe calprotectine-récepteur est formé, pour l'évaluation immédiate du taux calprotectine urinaire dans une méthode de différenciation entre une insuffisance rénale aiguë intrinsèque et prérénale.

11. Méthode selon l'une quelconque revendication 1 à 7, dans laquelle, si ledit patient est diagnostiqué avec une insuffisance rénale aiguë intrinsèque, on examine une biopsie rénale qui a été obtenue auprès dudit patient pour déterminer davantage la nature de l'insuffisance rénale aiguë et déterminer une thérapie appropriée.
